# EUROPEAN PATENT APPLICATION

(11) **EP 1 589 002 A1**
(43) Date of publication of application: **26.10.2005**
(21) Application number: 03788755.1
(22) Date of filing: 31.12.2003
(51) Int. Cl.: C07D 207/273, A61K 31/40, A61P 43/00

(54) **CLAUSENAMIDE C5 HYDROXYL DERIVATIVES AND N-SUBSTITUTED DERIVATIVES, PROCESSES FOR THEIR PREPARATION, ITS COMPOSITION AND USE**

(30) Priority: 31.12.2002 CN 02159343
(71) Applicant: THE INSTITUTE OF MATERIA MEDICA OF CHINESE ACADEMY OF MEDICAL SCIENCES, Xuan Wu Dist., Beijing 100050 (CN)
(72) Inventor: HUANG, Liang, Beijing 100050 (CN); ZHANG, Juntian, Beijing 100050 (CN); WU, Kemei, Beijing 100050 (CN); CHEN, Shiming, Beijing 100050 (CN); LI, Xingzhou, Beijing 100050 (CN)
(74) Representative: CAPRI
(86) International application number: PCT/CN2003/001150
(87) International publication number: WO 2004/058706

(57) **Abstract**

The present invention relates to stereo isomers of C₅-hydroxyl substituents, N-substituted derivatives of hydroxybenzyl, hydroxyl, phenyl substituted γ-lactams (clausenamide) and the preparation methods thereof, and pharmaceutical compositions containing the same and their uses for the preparation of medicaments as nootropic and anti-aging drugs.

## Description

### TECHNICAL FIELD

The present invention relates to clausenamide C₅-hydroxyl substituted stereo isomers, N-substituted derivatives and the preparation methods thereof, and pharmaceutical compositions containing the same and their uses for the preparation of medicaments as nootropic and anti-aging drugs.

### BACKGROUND ARTS

Up to now, the average life expectancy is over 70 in China, which is double of that of 1949 (founding of the People's Republic of China). A foreign research result suggests that the ratio of children under 15 will be 18.6% of total population statistics, and the ratio of old people of 65 or older against total population statistics will be 18.8%, which is over the ratio of children against total population statistics until 2025. The data shows that there will be 1 old people in every 5 people after two decades. Alzheimer's disease is often diagnosed in people older than 50. And multi-infarct dementia induced by cerebrovascular diseases or senile dementia is diagnosed in people over 60 years old. Therefore, the morbidity of Alzheimer's disease and senile dementia are likely to increase with the aging of population. Aged people with typical neural degeneration disorders (e.g. dementias) will experience two phases of death, the first is the death of soul, and followed by the death of body, which bring indescribable sorrow to the patients, and it is also a heavy burden to the society and families. Aging is regarded as one of the most riskful factors affecting the development and stability of the society only after war, murrain, famine and shortage of energy and resources.

There are a large number of drugs for the prevention of aging and treatment of senile dementia. Cerebrovascular dilation drugs can improve cerebral blood flow so as to take advantage of energy supply and cognition. However, a valuable vasodilator should be highly selective, not affecting cerebral metabolism, having no "blood-steal" effect, and also having anti-platelet aggregation and anti-thrombosis effects. Nimodipine, being a calcium antagonist, fits some of the above criteria, but it is only effective on L-type voltage-dependent calcium channel, and has no effect on N-type and T-type calcium channels. Among the drugs enhancing the function of cholinergic system, Ach-precursors only have moderate therapeutic effects, and Ach receptor agonists as well as cholinesterase inhibitors have certain effects, but the effects last shortly and the side effects as well as toxicity are relatively severe. A number of neuropeptides and nerve growth factors were considered to be the hope of dementia therapy, however, the data obtained from clinical research showed of little benefits to the patients, this probably because these substances can hardly pass through the blood-brain-barrier (BBB) to play a role in the brain. 2-Pyrrolidone acetamide (*piracetam*, which is being produced in China with the trade name of Nao-Fu-Kang), once was regarded as a novel nootropic drug which had no controversy in the earlier publications. In the recent years, it has been reported in China or abroad to be moderately or little effect on dysmnesia and senile dementia. One main reason is that being water-soluble, the drug has a low passing-through rate of BBB and can hardly concentrate on the target to be effective.

The present inventors firstly isolated a nootropic active agent from the plant *Clausena lansium (lour.)* Skeels of the family of *Rotaceae* containing the γ-lactam skeleton of piracetam and named it as Clausenamide. Clausenamide is a γ-lactam having four chiral centers, and is naturally presenting in the racemic form. The process for the preparation of (±) clausenamide was granted as EP0414020, the Chinese equivalent patents are CN86107090, ZL90107145.5 and ZL90107144.7. (-)Clausenamide shows effective nootropic activity, and the compound per se, its preparation and its potent pharmaceutical uses are disclosed in the pending Chinese patent application No. 00124630.5.

There are four chiral centers in the molecule of clausenamide and should have 16 stereoisomers, which form 8 pairs of respectively diasteric enantiomers, the configurations and nomenclatures thereof are shown in Table 1.

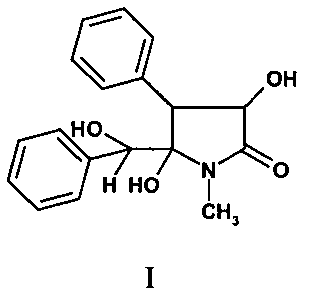

**Table 1,**

| 8 pairs of optical active stereo isomers | | |
|---|---|---|
| | Clausenamide (I₁) | Neoclausenamide (I₂) |
| | (-) 3S, 4R, 5R, 6S | (-) 3S, 4R, 5S, 6R |
| | (+) 3R, 4S, 5S, 6R | (+) 3R, 4S, 5R, 6S |
| C6 isomers | Epiclausenamide (I₃) | Epineoclausenamide (I₄) |
| | (+) 3S, 4R, 5R, 6R | (-) 3S, 4R, 5S, 6S |
| | (-) 3R, 4S, 5S, 6S | (+) 3R, 4S, 5R, 6R |
| C3 isomers (C3/C4, cis) | Cisclausenamide (I₅) | Cisneoclausenamide (I₆) |
| | (+) 3S, 4S, 5S, 6R | (-) 3S, 4S, 5R, 6S |
| | (-) 3R, 4R, 5R, 6S | (+) 3R, 4R, 5S, 6R |
| | Cisepiclausenamide (I₇) | Cisepineoclausenamide (I₈) |
| | (+) 3S, 4S, SS, 6S | (-) 3S, 4S, 5R, 6R |
| | (-) 3R, 4R, 5R, 6R | (+) 3R, 4R, 5S, 6S |

Among which, one pair of clausenamides (I₁) are disclosed in the pending Chinese patent application (Chinese patent application number 00124630.5). Racemic neoclausenamide (I₂) and epineoclausenamide (I₄) are known compounds, but the optical active neoclausenamide (I₂) and epineoclausenamide (I₄) and their preparation methods are unknown in the previous publications. *In vivo* and *in vitro* metabolic studies on (+) and (-) clausenamides (I₁) demonstrate that the metabolites of both compounds are substantially similar, while the contents of (-) N-hydroxymethyl norclausenamide [(-)CM₁] and (-) 5-hydroxyclausenamide [(-)CM₂] in the metabolites of (-) clausenamide are significantly higher than that of the corresponding (+) isomers (+)CM₁ and (+)CM₂ in the metabolites of (+) clausenamide. Pharmacological tests show that (-) clausenamide possesses effective nootropic activities, while (+) clausenamide does not possess such effect, but has antagonistic effects. Based on these discoveries, the enantiomers of the metabolites CM₁ and CM₂ and their derivatives were synthesized, and the synthesis of CM₁ has been published by the inventors.

The prior arts have not disclosed the synthesis of the optical active clausenamides and the derivatives thereof.

### SUMMERY OF THE INVENTION

An object of the present invention is to provide a novel series of clausenamide C₅-hydroxyl derivatives and N-substituted derivatives.

Another object of this invention is to provide preparation methods of the clausenamide C₅-hydroxyl derivatives and N-substituted derivatives.

One another object of the invention is to provide uses of the clausenamide C₅-hydroxyl derivatives and N-substituted derivatives for the preparation of medicaments as nootropic and anti-aging drugs.

The present invention relates to a clausenamide C₅-hydroxy derivative represented by general formula (II): 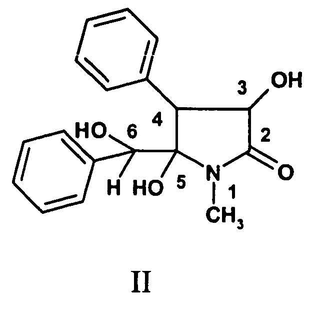 characterized that,
(rac)-II₁, (relative) configuration (3S*, 4S*, 5S*, 6R*); or
(rac)-II₂, configuration (3S*, 4S*, 5R*, 6S*); or
(rac)-II₃, configuration (3S*, 4S*, 5S*, 6S*).

Or, (+) II₁, (absolute) configuration: (3S,4S,5S,6R) or (-) II₁, configuration (3R,4R,5R,6S); or (+) II₂, configuration: (3S,4S,5R,6S), or (-) II₂, configuration: (3R,4R,5S,6R); or (+) II₃, configuration: (3R,4R,5R,6R) or (-) II₃, configuration: (3S,4S,5S,6S).

This invention also provides the preparation methods of the optical active clausenamide C₅-hydroxy derivatives represented by general formula (II), which is shown in the following **Scheme 1**. 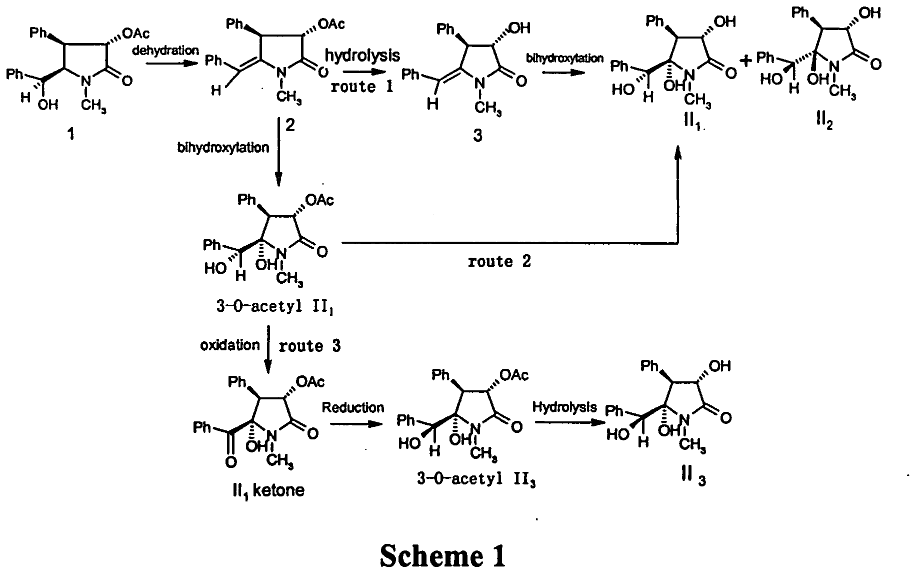
(1) Dehydrolation of (rac)-3-O-acetylclausenamide (1) or an optical isomer thereof, the dehydralating agent may be POCl₃/Py; alternatively, to prepare the methylsulfonate of clausenamide, then cleave the methylsulfonate group with DBU. The POCl₃/Py method is preferred.
(2) Hydrolysis of (rac)-3-O-acetyl-Δ^{5,6}-clausenamide (2) can be carried out under conventional acid or base conditions.
(3) Bihydroxylation of Δ^{5,6}-clausenamide (3) can be achieved via OsO₄/NMO, KHSO₅/CH₃COCF₃, WO₃/H₂O₂, etc. OsO₄/NMO method is preferred.
(4) Oxidation of (3S*, 4S*, 5S*, 6R*)-3-O-acetyl-5-hydroxy clausenamide (3-O-acetyl II₁) or an optical active isomer thereof may be carried out with the oxidants such as KMnO₄/CuSO₄, MnO₂, DMSO/ClCOCOCl/TEA, DMSO/trifluroacetic anhydride (TFAA)/TEA, etc, preferably DMSO/ClCOCOCl/TEA.
(5) Reduction of 3S*, 4S*, 5S*)-3-O-acetyl-5-hydroxy clausenamidone (II₁ ketone) or an optical active isomer thereof can be carried out via various borohydrides, preferably sodium borohydride, and lithium tri-sec-butyl borohydride.
(6) Hydrolysis of (3S*, 4S*, 5S*, 6S*)-3-acetoxy-5-hydroxy clausenamide (II₃) or an optical active isomer thereof may be carried out under acid or base conditions, or Sm(samarium)/I₂/CH₃OH, preferably Sm/I₂/CH₃OH.

The present invention also relates to a racemic or optical active isomer of N-substituted derivative of clausenamide, which is represented by general formula (III): 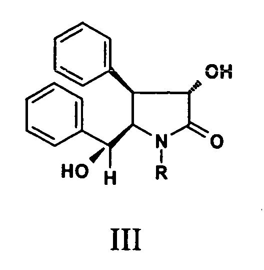 Wherein the relative configuration is (3S*,4R*,5R*,6S*) and subsutituent R is selected from CH₂COR¹, CH₂OCH₂COR², and CH₂R³,
wherein,
R₁ is selected from OH, NH₂, C₁₋₈ alkoxy, 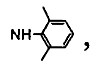 and 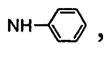 R₂ is selected from C₁₋₈ alkoxy, and
R₃ is selected from 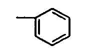 and

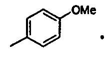

The preparation methods of the N-substituted clausenamide derivatives, when R is selected from CH₂COR¹, CH₂OCH₂COR², are shown in **Scheme 2.**

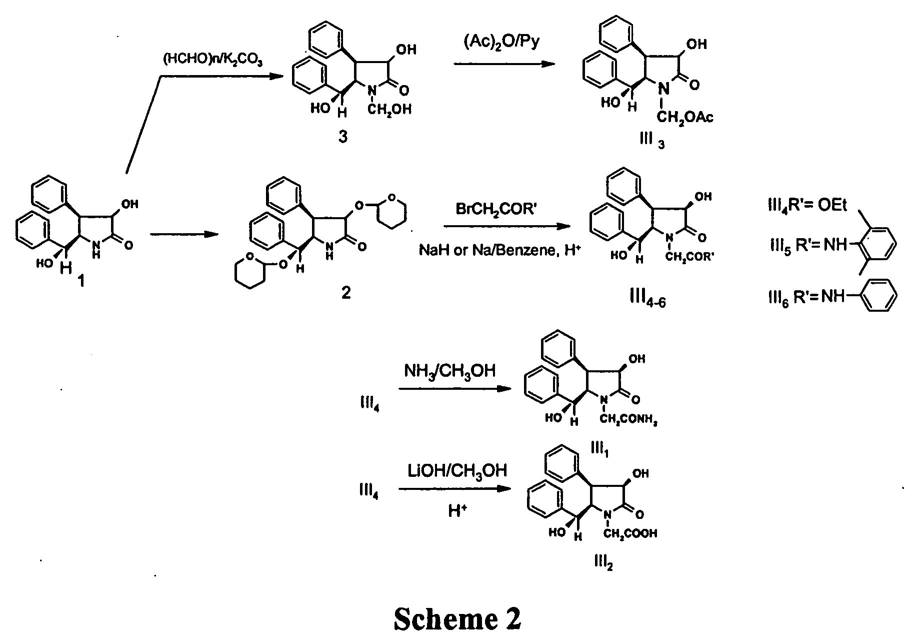

Reacting norclausenamide (1) with dihydropyran under the catalysis of pyridinium *p*-toluenesulfonate to give 3,6-di-O-tetrahydropyran-norclausenamide (2). 3,6-Di-O-tetrahydropyran-norclausenamide (2) is dissolved in anhydrous benzene, adding sodium hydride and then the mixture is heated in oil-bath for half an hour, followed by stirring at room temperature for 10 minutes. Anhydrous benzene solution of bromoacetate is added, and the reaction is carried out at 60°C in oil-bath for 20 minutes, then the reaction solution is allowed to return room temperature and water is added and stirred. The mixture is diluted with methylene chloride, adjusted to neutral with hydrochloric acid and the organic layer is separated, and washed and dried., The solvent is evaporated to obtain a oily substance, which is dissolved in ethanol and 10mg of p-toluene sulfonic acid is added. The tetrahydropyran protection groups are cleaved at 60°C in oil-bath to release the protection group of tetrahydopyran. After the reaction is completed, the compounds are directly purified by column chromatography to achieve N-(alkoxy/alkylaminocarbonylmethylene) norclausenamides (III₄).

Treating 60 mg of N-(ethoxycarbonylmethylene)norclausenamide (III₄) with a largely excess amount of NH₃/CH₃OH solution and the mixture is laid over night. After column chromatography purification, N-(aminocarbonylmethylene)norclausenamide (III₁) is obtained.

Dissolving norclausenamide (1) in acetone and adding water dropwisely, then paraformaldehyde and potassium carbonate are added. The mixture is heated to an outer-temperature of 60°C. After 25 minutes, stop heating and quickly cooling the reaction mixture with ice-water, evaporating the solvent under reduced pressure to afford a sticky liquid. N-(hydroxylmethly)norclausenamide (3) is obtained upon column chromatography. N-(hydroxymethly)norclausenamide (3) is dissolved in anhydrous tetrahydrofuran (THF) and stirred in an ice-salt bath for 20 minutes, and anhydrous THF solution of the corresponding anhydride and pyridine is added dropwisely and stirred at the temperature for 3 hours. A small amount of water is added and diluting the mixture with methylene chloride. Organic layer is separated and washed. Corresponding N-(acyloxymethylene)-norclausenamide is obtained by evaporating the solvent and followed by a column chromatography.

The N-substituted clausenamide derivatives as represented by general formula III when R is selected from CH₂R³ can be prepared from the reduction of the intermediates of CM₁, N-benzyl- or -N-p-methoxybenzyl-clausenamidone (as shown in **Scheme 3):**

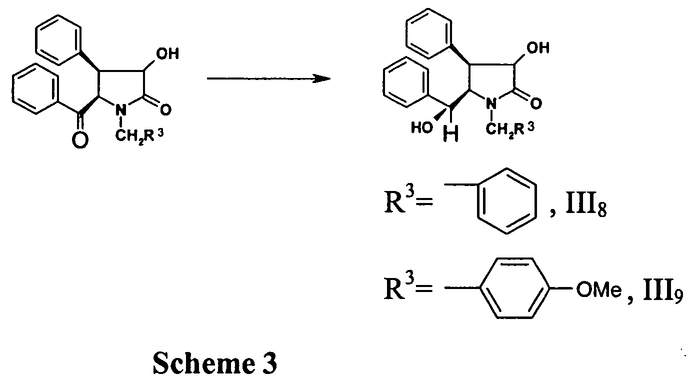

The present invention also relates to a pharmaceutical composition comprising the present compounds as an active ingredient and a normal pharmaceutically acceptable carrier or excipient. Generally, the present pharmaceutical composition may contain 0.1~95% by weight of a present compound.

The present pharmaceutical composition may be prepared via ordinary methods commonly known in the art. For such purposes, if necessary, the present compound may be combined with one or more solid or liquid pharmaceutical carriers and/or excipients, to prepare an administration form or dosage form suitable for human or veterinary uses.

The present compounds or the pharmaceutical composition comprising the same may be administered in a unit dosage form. And the administration route may be intestinal or parenteral, such as oral, intramuscular, substcutaneous, nasal, buccal, transdermally, intraperitoneally, or rectal, preferably oral.

The present compounds or the pharmaceutical composition comprising the same may be administered via injection, which may be *i*.*v*., *i*.*m*., substcutaneous or intradermic injections.

The formulation may be a normal form, a sustained release form, a controlled release form, a targeting release form or various microgranule delivery systems.

Various carriers commonly used in the art can be used to prepare tablets. Carriers such as, diluents and absorbents, for example, starch, dextrin, calcium sulfate, lactose, mannitol, sucrose, sodium chloride, glucose, urea, calcium carbonate, China clay, microcrystallinecellulose, aluminum silicate, etc; moistening agents and adhesives, for example, water, glycerol, polyethylene glycol, ethanol, propanol, starch paste, dextrin, syrup, honey, glucose solution, Arabia gum, gelatin liquid, carboxymethylcellulose sodium, lac, methylcellulose, potassium phosphate, polyvinylpyrrolidone, etc; disintegrants, for example, dry starch, alginate, agar powder, algin starch, sodium bicarbonate and citric acid, calcium carbonate, polyethylenesorbitol fatty acid ester, sodium dodecanylsulfonate, methylcellulose, ethylcellulose, etc; disintegration inhibitors, for example, sucrose, glycerin tristearate, cocoa butter, hydrogenated oils, etc; sorbefacients, for example, quaternary ammonium salts, sodium dodecanylsulfonate, etc; lubricants, for example, talc, silica, corn starch, stearates, boric acid, liquid paraffin, polyethylene glycol, etc. The tablets can be further coated, for example sugar-coated, thin film-coated, enteric-coating coated, or bi-layered or multi-layered tablets.

Carriers commonly used in the art can be used for the preparation of pellets from dosage units, such as, diluents and absorbants, for example, glucose, lactose, starch, cocoa butter, hydrogenated vegetable oil, polyvinylpyrrolidone, Gelucire, Kaolin, talc, etc; adhesives, for example, Arabia gum, tragacanth gum, gelatin, ethanol, honey, sugar liquid, rice paste or flour paste, etc; disintegrants, for example, agar powder, dry starch, alginate, sodium dodecanylsulfonate, methylcellulose, ethylcellulose, etc.

To prepare capsules from dosage units, the compounds of the invention are admixed with the above-mentioned carriers and the mixtures are fixed into hard gelatin capsules or soft capsules. The active ingredients, compounds of the present invention, can also be encapsuled into microcapsules, which can be suspended an aqueous medium to prepare a suspension, or be fixed into hard capsules or prepared into injections.

To prepare injections such as solution, suspension, emulsion, and lyophilized powder for injection, which can be hydrous or anhydrous and may comprise one or more pharmaceutically acceptable carriers, diluents, adhesives, moistening agents, lubricants, preservatives, surfactants, and dispersants. Such as diluents may be water, ethanol, polyethylene glycol, trimethylene glycol, ethoxylated isostearol, multioxygenated isostearol, polyethyleneoxide sorbitol alkanates, etc. In addition, an appropriate amount of sodium chloride, glucose or glycerol can be added into injections to produce isotonic injections. Moreover, conventional solubilizing agents, buffers, and pH modulators can also be added. These are all commonly known by those skilled of the art.

In addition, if necessary, colorants, antiseptics, fragrants, flavoring agents, sweetening agents and the other materials can be added into the dosage forms.

The drug or pharmaceutical composition can be administered by any commonly known method to achieve the treatment object and enhance the therapeutic effects.

The administration dosage of the compounds of the present invention or a pharmaceutical composition thereof varies, depending on several factors, for example, the characters and severeness of disorders to be prevented or treated, gender, age, weight, and individual reaction of patients, on the concrete conpound, administration route and frequency, etc. A suitable dosage of the present compound is within an amount of from 0.001mg/kg to 150 mg/kg body weight per day, preferably from 0.01mg/kg to 100 mg/kg body weight per day, more preferably from 0.01mg/kg to 60 mg/kg body weight per day, and most preferably from 0.1mg/kg to 10 mg/kg body weight per day, which can be administered once daily or severally divided (such as twice, three time or four times), depending on the physician's decision and other therapeutic means.

The total dosage for each therapeutic means may be administered once or severally divided, and the compounds and compositions of present invention may be administered singly or in combination with other therapeutic drugs, in the latter circumstance, the dosage may be adjusted accordingly.

In the assay of clone-forming rate of human embryo-neural stem cells, the present compounds showed effective enhancement on the forming of human embryo-neural stem cell clones, which illustrates that the present compounds possess effects on treating senile neurodegenerative diseases such as senile dementia.

In the assay of long-term potentiation (LTP) in rat hippocampus, the present compounds showed effective enhancement on LTP. Moreover, the compounds of present invention were found to have enhancing effect on basic synaptic transmission in neurons, indicating the nootropic effects of the compounds.

### EXAMPLES

The following examples are used to further describe the invention, but not intended to limit the invention at any way.

### Example 1

### Preparation of racemic (3S*,4S*,5R*,6S*)-5-hydroxyclausenamide (II₂) (route 1)

1.00 g Racemic 3-O-acetyl-clausenamide (1) was dissolved in 6ml anhydrous pyridine under a dry condition, cooled in an ice-water bath for 20 minutes, followed by addition of a solution of 1.0 ml distilled phosphorous oxychloride in 4ml anhydrous pyridine dropwise over half an hour, the stirring continued in ice-water bath for 24 hours. The whole mixture was poured into ice water. After solids were precipitated completely, the solids were separated by filtration. The filter cake was dissolved with methylene dichloride, washed with water and sodium chloride solution successively, dried over anhydrous sodium sulfate, and evaporate off solvents to give a solid. The solid was separated by column chromatography to give 746 mg racemic 3-O-acetyl-Δ^{5,6}-clausenamide (2), white solid, yield: 71% , mp: 148-150°C.

400 mg racemic 3-O-acetyl-Δ^{5,6}-clausenamide (2) was dissolved in 15 mL methylene dichloride, to the mixture was added 3 mL 10% sodium hydroxide alcohol solution, after 3 minutes, the mixture was neutralized with 2mol/L HCl to neutrality, diluted with 20 mL methylene dichloride, washed with 1 mol/L sodium bicarbonate solution and sodium chloride solution, dried over anhydrous sodium sulfate. The solvents were evaporated to give 330 mgΔ^{5,6}-clausenamide (3), white solid, yield: 95%, mp: 162-163°C.

260 mg Δ^{5,6}-clausenamide (3) was dissolved in 1 ml acetone and 5 ml THF, to which were added 545 mg N-oxo-N-methyl-morpholine and 0.6ml of 4% OsO₄ aqueous solution with a syringe, stirred at room temperature for 24 hours, to the mixture were added 650 mg anhydrous sodium sulfite and 3 ml water, stirred for 1 hour, diluted with methylene dichloride. The organic layer was separated. The aqueous layer was extracted with methylene dichloride, the organic phases were combined, washed with sodium chloride aqueous solution, dried over anhydrous sodium sulfate, and evaporated off solvents to give 280 mg oily residue, which was purified by chromatography to give 150 mg (±)-(3S*,4S*,5R*,6S*)-5-hydroxy- clausenamide (II₂), white solid, mp:134-136°C, yield: 50%, and 70 mg (±)-(3S*, 4S*,5S*,6R*)-5-hydroxyclausenamide (II₁) as a by-product, white solid, mp: 127-129°C, yield: 24%.

### Example 2

### Preparation of (±)-(3S*,4S*,5S*,6R*)-5-hydroxy-clausenamide (II₁) (route 2)

240 mg racemic 3-O-acetyl-Δ^{5,6}-clausenamide (2) was dissolved in 4ml THF/acetone (5/1), to which were added 581 mg N-oxo-N-methyl-morpholine and 1.9ml 10mg/ml OsO₄ aqueous solution, stirred at room temperature for 48 hours, to the mixture were added 500 mg anhydrous sodium sulfite and 10 ml water, stirred for 1 hour, transferred to a separating funnel and washed with methylene dichloride. The organic layer was washed with sodium chloride aqueous solution, dried over anhydrous sodium sulfate, and distilled off solvents to give 300 mg oily residue, which was recrystallized with ethyl acetate and separated with preparative thin-layer-chromatography (TLC) (ethyl acetate) to give 217 mg (±)-(3S*,4S*,5S*, 6R*)-5-hydroxy-clausenamide (II₂), white solid, mp:134-136°C, yield: 50%, and 70 mg (±)-(3S*,4S*,5S*,6R*)-3-O-acetyl-5-hydroxy-clauscnamide (3-O-acetyl-II₁), yield: 82%, mp:163-165°C.

(±)-(35*,45*,55*,6R*)-3-O-acetyl-5-hydroxy-dausenamide (3-O-acetyl-II₁) was deacetylated with Sm/I₂/CH₃OH to yield (±)-(3S*,4S*, 5S*,6R*)-5-hydroxy-clausenamide (II₁), mp: 127-129°C. The concrete operations are as described in the last step hydrolysis in example 7.

Preparation of optical active (3S,4S,5R,6S)-5-hydroxy-clausenamide (II₂), (3R,4R,5S,6R)-5-hydroxy-clausenamide (II₂), (3S,4S,5S,6R)-5-hydroxy-clausenamide (II₁), (3R,4R,5R,6S)-5-hydroxy-clausenamide(II₁).

Same as the methods described for the preparation of racemic compounds above, except that the starting material being optical active 3-O-acety-clausenamide (1). The yields in each step were similar to those in the preparation of racemic compounds. The melting points and [α] values of the products and intermediates are listed as following.
(-)-3-O-acetyl-Δ^{5,6}-clausenamide, mp:119-120°C, [α]_{D}¹⁸=-330(c, 0.870, CHCl₃)
(+)-3-O-acetyl-Δ^{5,6}-clausenamide,mp:119-120°C,[α]_{D}¹⁸=+326(c, 0.870, CHCl₃)
(-)-Δ^{5,6}-clausenamide, mp:125-127°C,[α]_{D}¹⁸=-17.1 (c, 0.700, CHCl₃)
(+)-Δ^{5,6}-clausenamide, mp:125-127°C, [α]_{D}¹⁸=+16.7 (c, 0.738, CHCl₃)
(+)-(3S,4S,5S,6R)-5-hydroxy-clausenamide, oil, [α]_{D}²⁵=+113 (c, 0.505, CH₃OH)
(-)-(3R,4R,5R,6S)-5-hydroxy-clausenamide, oil, [α]_{D}²⁵=-117 (c, 0.200, CH₃OH)
(+)-(3S,4S,5R,6S)-5-hydroxy-clausenamide, mp:147-149°C, [α]_{D}²⁵= +202 (c, 0.510, CH₃OH)
(-)-(3R,4R,5S,6R)-5-hydroxy-clausenamide, mp: 145-147°C, [α]_{D}²⁵= -208 (c, 0.620, CH₃OH)
(-)-(3S,4S,5S,6R)-3-O-acetyl-5-hydroxy-clausenamide (3-O-acetyl II₁), mp: 125-128°C, [α]_{D}¹⁵=-323 (c, 0.870, CH₃OH)
(+)-(3R,4R,5R,6S)-3-O-acetyl-5-hydroxy-lausenamide (3-O-acetyl II₁), mp: 125-127°C,[α]_{D}¹⁵=+327 (c, 0.470, CH₃OH)

### Example 3

### A: Preparation of racemic (±)-(3S*,4S*,5S*,6S*)-5-hydroxy-clausenamide (II₃) (route 3).

To a dry three-necked flask 5 mL THF was added and cooled to -50~-60°C. 453µl Re-distilled oxalyl chloride was added under an atmosphere of N₂, stirred for 1 min, 767µl dimethyl sulfoxide (DMSO) (dried with molecule sieve) was added slowly at -50~-60°C, stirred for 5 min, 10 ml THF solution of 150 mg (±)-(3S*,4S*,5S*,6R*)-3-O-acetyl-5-hydroxy-clausenamide (3-O-acetyl II₁) was added slowly. After completion of addition, the resultant mixture was stirred for 2 hours at the temperature and then 1ml triethylamine was added. Kept at the temperature for 0.5 h and then warmed slowly to room temperature. 10 ml water was added, and THF was distilled off under reduced pressure. The residue was extracted with ethyl acetate until no product detectable in the aqueous layer. The organic layer was washed with 1 mol/l hydrochloric acid, aqueous sodium bicarbonate, saturated sodium chloride solution, and dried over magnesium sulfate, distilling off solvent to give 250 mg oil. Purification by column chromatography gave 95 mg (±)-(3S*,4S*,5S*)-3-O-acetyl-5-hydroxyclausenamidone (3-O-acetyl II₁ ketone), yield: 71%.

82 mg (±)-(3S*,4S*,5S*)-3-O-acetyl-5-hydroxyclausenamidone (3-O-acetyl II₁ ketone) was dissolved in 3ml methanol, stirred in an ice-water bath for 20 min. To the mixture was added 30 mg sodium borohydrate, stirred for 10 min in the ice-water bath. The reaction mixture was neutralized carefully with 1 mol/l hydrochloric acid and then a small amount of water was added. The organic solvents were distilled off under reduced pressure. The residue was dissolved with ethyl ether, the ether layer was separated, and the aqueous layer was washed with ethyl ether. The ether layers were combined, and washed with sodium chloride aqueous solution, dried over anhydrous sodium sulfate, and distilled off solvent to give an oil. Purification by column chromatography gave 60 mg (3S*,4S*,5S*,6S*)-3-O-acetyl-5-hydroxyclausenamide (3-O-acetyl-II₃), mp:144-147°C, yield: 73%.

50mg (3S*,4S*,5S*,6S*)-3-O-acetyl-5-hydroxyclausenamide (3-O-acetyl-II₃) was dissolved in 50ml anhydrous methanol, to the mixture was added 38 mg iodine and 23 mg samarium. The resultant mixture was stirred in air for 12 hours, diluted with ethyl acetate and transferred to a separating funnel, washed with 25% sodium hyposulfite solution, the organic layer was separated, washed with sodium chloride solution, dried over anhydrous sodium sulfate, distilled off solvents under reduced pressure, and purified by column chromatography to give 25 mg (+)-(3S*,4S*,5S*,6S*)-5-hydoxy-clausenamide(II₃), which is recrystallized with ethyl acetate/ petroleum ether to give a white solid, mp:114-116°C, yield: 56%.

### B: Preparation of (3S,4S,5S,6S)-5-hydoxy-clausenamide (II₃) and (3R,4R,5R,6R)-5-hydoxy-clausenamide(II₃).

Same as the preparation methods of the racemic compounds as described in A, using (-)-(3S,4S,5S,6R)-3-O-acetyl-5-hydroxyclausenamide (3-O-acetyl-II₁) or (+)-(3R,4R,5R,6S)-3-O-acetyl-5-hydroxyclausenamide (3-O-acetyl-II_{I}) as starting materials, the compounds were synthesized with the yields in each step identical with those in the preparation of racemic compounds. The melting points and rotatory values of the compounds were listed as following:
(-)-(3S,4S,5S)-3-O-acetyl-5-hydroxy-clausenamidone (3-O-acetyl-II₁ ketone), mp:125-128°C, [α]_{D}¹⁵=-310 (c, 0.360, CHCl₃)
(+)-(3R,4R,5R)-3-O-acetyl-5-hydroxy-clausenamidone (3-O-acetyl-II₁ ketone), mp:127-129°C, [α]_{D}¹⁸= +312 (c, 0.442, CHCl₃)
(-)-(3S,4S,5S,6S)-3-O-acetyl-5-hydroxy-clausenamide (3-O-acetyl-II₃), mp: 153-157°C, [α]_{D}¹⁵= -31.9 (c, 0.455, CH₃OH)
(+)-(3R,4R,5R,6R)3-O-acetyl-5-hydroxy-clausenamide (3-O-acetyl-II₃), mp:152-156°C, [α]_{D}¹⁸= +31.7 (c, 0.480, CH₃OH)
(-)-(3S,4S,5S,6S)-5-hydroxy-clausenamide, oil, [α]_{D}¹⁸=-53.6 (c, 0.470, CH₃OH)
(+)-(3R,4R,5R,6R)-5-hydroxy-clausenamide, oil, [α]_{D}¹⁸=+54.1 (c, 0.480, CH₃OH)

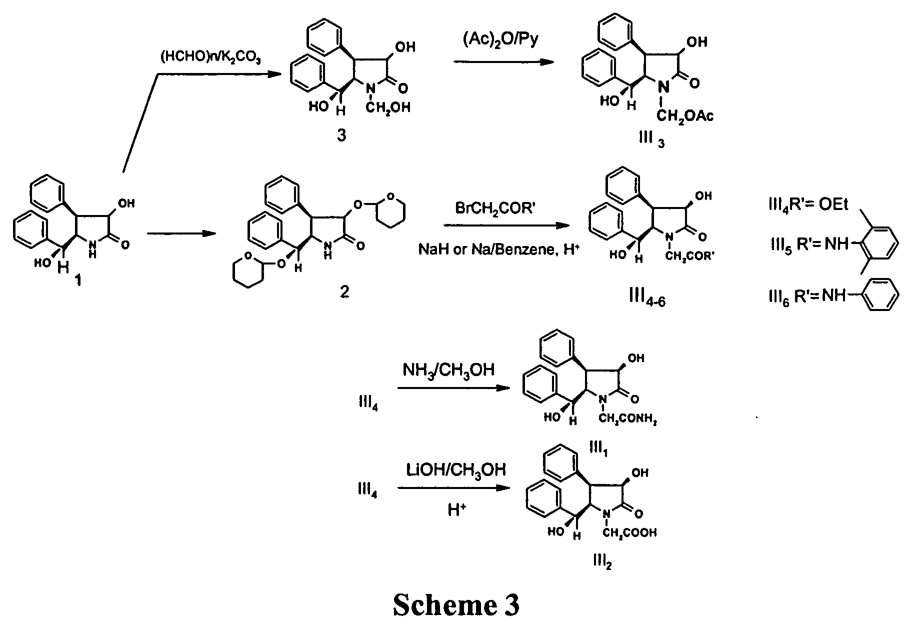

### Example 4: Preparation of N-(ethoxycarbonyl-methylene)-norclausenamide (III₄) (as shown in Scheme 3)

### (1) Preparation of optical active 3,6-di-O-tetrahydropyranyl-neoclausenamidone

443mg (-)-(3R,4S,SR)-neoclausenamidone (1.5mmol), 280 mg 3,4-dihydropyran (4.5 mmol) and 38 mg pyridinium p-toluenesulonate (0.15mmol) were stirred over night, diluted with 10mL methylene dichloride, washed with aqueous sodium chloride, dried over anhydrous sodium sulfate, and concentrated to give 0.51g (+)-3,6-di-O-tetrahydropyranyl-neoclausenamidone, yield: 89.6%, mp: 181-7°C, [α]_{D}¹⁵ +13.46 (c, 0.42, CHCl₃).

416mg (-)=3,6-di-O-tetrahydropyranyl-neoclausenamidone was obtained with 0.35g (+)-(3S,4R,5S)-neoclausenamidone as starting material by the process as described above, yield: 91.4%, mp: 194-200°C, [α]_{D}¹⁵ -11.9 (c, 0.24, CHCl₃).

120 mg 3,6-di-O-tetrahydropyranyl-norclausenamide (2) was dissolved in 5ml anhydrous benzene, 21mg sodium hydride was added. The mixture was heated in oil-bath for half an hour and then stirred at room temperature for 10 minutes, added a solution of 66 mg ethyl bromoacetate in 5ml dry benzene, heated at 60°C in oil-bath for 20 minutes, cooled the reaction solution to room temperature, added 3ml water and stirred, diluted with methylene chloride, and neutralized with 2N hydrochloric acid. The organic layer was separated, washed with sodium chloride aqueous solution, dried over anhydrous sodium sulfate, distilled off solvents to give oily product. The oily product was dissolved in ethanol, treated with 10mg p-toluenesulfonic acid, then heated at 60°C in oil-bath to strip off the tetrahydropyran moiety, after the reaction was completed, the product was directly purified by column chromatography to give the title compound, 79mg, oil, yield 82%.

### Example 10: Preparation of N-(aminocarbonyl-methylene)-norclausenamide (III₁)

60 mg N-(ethoxycarbonyl-methylene)-norclausenamide (III₄) was treated with greatly excess NH₃/CH₃OH solution, laid over night, purified by column chromatography to give 40 mg of the title compound, white solid, mp: 146-148°C, yield 72%.

### Example 5: Preparation of N-acetoxymethylene-norclausen amide (III₃).

200 mg Norclausenamide (1) was dissolved in 7.5 ml acetone, added 3 drops of water, then added 33mg paraformaldehyde and 9mg potassium carbonate. The reaction mixture was heated to an outer temperature of 60°C, and stopped heating after 25 minutes, quickly cooled in ice-water, evaporated off the solvent under reduced pressure to give 208 mg slurry, which was purified by column chromatography to give 162mg N-hydroxymethyl-norclausenamide (3), white solid, mp: 199-201°C, yield: 73%.

155mg N-hydroxymethylnorclausenwnide (3) was dissolved in 10ml dry THF, stirred in an ice-salt bath (-8°C) for 20 minutes, and added dropwise 75mg acetic anhydride and 59mg pyridine in 2ml dry THF, stirred at the temperature for 3 hours, added a small amount of water, diluted with methylene chloride. The organic layer was separated, washed each once with water and sodium chloride aqueous solution, dried over anhydrous sodium sulfate, distilled off solvent, and purified by column chromatography to give N-acetoxymethylene-norclausenamide (III₃) 60mg, mp: 137-139°C, yield: 32%.

### Pharmacological Tests

### Experimental example 1 Effects of stereo isomers of C5-hydroxyl substituted clausenamides and N-substituted clausenamide derivatives on cell proliferation of mouse brain neural stem cells.

### 1. Aim and significance

The increased number or prolonged lives of neural stem cells are of great importance for the increment of neuron and prevention of neuron lost. Mouse brain neural stem cells were used for evaluating cell proliferation effects of C5-hydroxyl substituted clausenamides and N-substituted clausenamide derivatives.

### 2. Method

C 17-2 mouse brain neural stem cells (C17-2 cells) were continuously cultured in 1640 medium with 10% horse serum and 10% fetal bovine serum at 5% CO₂ and 37°C, dissociated to single cell suspension by 0.1% trypsin solution, centrifuged at 1, 000 rpm × 5min and rinsed with medium. The cells were seeded in a 96-well plate, 1 × 10³ cells per well, 3 well per group, continuously cultured. After 24 hours, the test compounds were added, doses of each of the C5-hydroxyl substituted clausenamides and N-substituted clausenamide derivatives were 0.1µM, 1*µ*M and 10*µ*M respectively. The growth situation of the cells was observed under a microscope, once daily. At the 4^{th} day, media in each well were pipetted, phosphate buffer solution containing MTT was added. 4 Hours later, the fluid was discarded, and a DMSO solution was added. The cells were incubated at 37°C for 10 min under shaken. OD values on 490 nm of each well were detected. Set the growth rate of the control group as 100%, and the growth rates of each dose were given by comparison with the control group and t-tested.

### 3. Results

Table 1 shows the evaluation results of the C5-hydroxyl substituted clausenamides and N-substituted clausenamide derivatives on cell proliferation of mouse brain neural stem cells.

Among the 16 compounds, two C5-hydroxyl substituted clausenamide stereo isomers (Nos. 5, (-)II₁ (C₁₈H₁₉NO₄313.36) and 8, (+)II₃ (C₁₈H₁₉NO₄ 313.36)) and one N-substituted clausenamide derivative (No. 12, (±)III₅ (C₂₇H₂₈N₂O₄ 444.54) showed significant promotion effects on cell proliferation of mouse brain neural stem cells at doses of 0.1 µM and 1 µM.

**Table 1.**

| **Effects of Stereo Isomers of C5-hydroxyl Substituted Clausenamides and N-substituted Clausenamide Derivatives on Cell Proliferation of Mouse Brain Neural Stem Cells** | | | | | | |
|---|---|---|---|---|---|---|
| No. | Compounds | Formula | M.W. | Dose | Result (OD value) | Cell proliferation % |
| N.C. | DMSO | | | 0 | 0.137±0.021 | 100.00 |
| P.C. | FGF | | | 5mg/L | 0.340±0.035** | 248.17 |
| 1 | (-)Δ^{5,6}-I | C₁₈H₁₇NO₂ | 279.34 | 10⁻⁵ M | 0.076±0.016 | 55.47 |
| | | | | 10⁻⁶ M | 0.165±0.011 | 120.44 |
| | | | | 10⁻⁷M | 0.246±0.005 | 179.56 |
| 2 | (-)II₁ | C₁₈H₁₉NO₄ | 313.36 | 10⁻⁵ M | 0.126±0.016 | 91.97 |
| | | | | 10⁻⁶ M | 0.236±0.049* | 172.26 |
| | | | | 10⁻⁷ M | 0.256±0.020** | 186.86 |
| 3 | (+)II₁ | C₁₈H₁₉NO₄ | 313.36 | 10⁻⁵ M | 0.093±0.007 | 67.88 |
| | | | | 10⁻⁶ M | 0.264±0.036* | 192.70 |
| | | | | 10⁻⁷ M | 0.250±0.016** | 182.48 |
| 4 | (-)-3-O-AC-II₁ | C₂₀H₂₁NO₅ | 355.39 | 10⁻⁵ M | 0.063±0.014 | 45.99 |
| | | | | 10⁻⁶ M | 0.072±0.006 | 52.55 |
| | | | | 10⁻⁷ M | 0.179±0.004 | 130.66 |
| 5 | (-)II₂ | C₁₈H₁₉NO₄ | 313.36 | 10⁻⁵ M | 0.156±0.095 | 113.87 |
| | | | | 10⁻⁶ M | 0.163±0.038 | 118.98 |
| | | | | 10⁻⁷ M | 0.233±0.033 | 170.07 |
| 6 | (+)II₂ | C₁₈H₁₉NO₄ | 313.36 | 10⁻⁵ M | 0.076±0.008 | 55.47 |
| | | | | 10⁻⁶ M | 0.220±0.023 | 160.58 |
| | | | | 10⁻⁷ M | 0.175±0.059 | 127.74 |
| 7 | (-)II₃ | C₁₈H₁₉NO₄ | 313.36 | 10⁻⁵ M | 0.088±0.006 | 64.23 |
| | | | | 10⁻⁶ M | 0.187±0.020 | 136.50 |
| | | | | 10⁻⁷ M | 0.168±0.076 | 122.63 |
| 8 | (+)II₃ | C₁₈H₁₉NO₄ | 313.36 | 10⁻⁵ M | 0.131±0.009 | 95.62 |
| | | | | 10⁻⁶ M | 0.199±0.024 | 145.26 |
| | | | | 10⁻⁷ M | 0.253±0.020* | 184.67 |
| 9 | (±)III₁ | C₁₉H₂₀N₂O₄ | 340.38 | 10⁻⁵ M | 0.104±0.009 | 75.91 |
| | | | | 10⁻⁶ M | 0.205±0.006 | 149.64 |
| | | | | 10⁻⁷ M | 0.240±0.017* | 175.18 |
| 10 | (±)III₂ | C₁₉H₁₉NO₅ | 341.37 | 10⁻⁵ M | 0.100±0.018 | 72.99 |
| | | | | 10⁻⁶ M | 0.175±0.006 | 127.74 |
| | | | | 10⁻⁷ M | 0.218±0.012 | 159.12 |
| 11 | (-)III₃ | C₂₀H₂₁NO₅ | 355.39 | 10⁻⁵ M | 0.061±0.007 | 44.53 |
| | | | | 10⁻⁶ M | 0.147±0.021 | 107.30 |
| | | | | 10⁻⁷ M | 0.217±0.024 | 158.39 |
| 12 | (±)III₅ | C₂₇H₂₈N₂O₄ | 444.54 | 10⁻⁵ M | 0.171±0.035 | 124.82 |
| | | | | 10⁻⁶ M | 0.227±0.011* | 165.69 |
| | | | | 10⁻⁷ M | 0.304±0.051** | 221.90 |
| 13 | (±)III₆ | C₂₅H₂₄N₂O₄ | 416.48 | 10⁻⁵ M | 0.147±0.051 | 107.30 |
| | | | | 10⁻⁶ M | 0.163±0.003 | 118.98 |
| | | | | 10⁻⁷ M | 0.230±0.013 | 167.88 |
| 14 | (±)III₇ | C₂₄H₂₁NO₃ | 371.44 | 10⁻⁵ M | 0.146±0.011 | 106.57 |
| | | | | 10⁻⁶ M | 0.196±0.007 | 143.07 |
| | | | | 10⁻⁷ M | 0.219±0.017 | 159.85 |
| 15 | (±) III₈ | C₂₄H₂₃NO₃ | 373.46 | 10⁻⁵ M | 0.096±0.022 | 70.07 |
| | | | | 10⁻⁶ M | 0.144±0.038 | 105.11 |
| | | | | 10⁻⁷ M | 0.221±0.043 | 161.31 |
| 16 | (±) III₉ | C₂₅H₂₅NO₄ | 403.48 | 10⁻⁵ M | 0.120±0.027 | 87.59 |
| | | | | 10⁻⁶ M | 0.138±0.012 | 100.73 |
| | | | | 10⁻⁷ M | 0.212±0.011 | 154.74 |
| M.W., Molecular weight | | | | | | |
| N.C., Negative control | | | | | | |
| P.C., Positive control | | | | | | |
| FGF, fibroblast growth factor | | | | | | |

### 4. Conclusion

The stereo isomers of C5-hydroxyl substituted clausenamides and N-substituted clausenamide derivatives of present invention show effects on promotion on cell proliferation of mouse brain neural stem cells. Among the compounds, two C5-hydroxyl substituted clausenamide stereo isomers (Nos. 5, (-)II₁ and 8, (+)II₃) and one N-substituted clausenamide derivative (No. 12, (±)III₅) show significant effects on cell proliferation of mouse brain neural stem cells.

### Experimental example 2 Effects of stereo isomers of C5-hydroxyl substituted clausenamides and N-substituted clausenamide derivatives on LTP induced in rat hippocampus

### 1. Aim and Significance

Significant nootropic effect of clausenamide was observed in several behavioral studies. But its influence on synaptic transmission remains under estimation. Synapses are the fundamental unit of message processing and transferring from cell to cell in nervous system, and alteration of the structure, activity and function of synapses is the neurobiological basis for learning and memory studies. Therefore, the nootropic effect of clausenamide was studied on synaptic level by using electrophysiological technology.

### 2. Method

Male adult SD Rats (5 per group) were anesthetized with 20% (w/v) urethane carbamate (1.0g·kg⁻¹, ip) and fixed in a stereotaxic head holder. According to the reference of Pellegrino stereotaxic spectrum of rat, recording electrodes (stainless steel needle of 0.2mm diameter were insulted except for 0.2mm tip) were placed in the dentate gyrus granule cell layer. Four pairs of optically active clausenamides were subjected into lateral ventricle as shown in table 9. The stimulating electrodes (two stainless steel needles of 0.15mm diameter coated with Teflon except for the 0.2mm tip, and the distance between two needles was 0.5mm) were planted between the fibre of perforant path (PP) in rat. Continuous pulse was provided by electronic stimulator, and delivered to PP through stimulus isolator and stimulating electrodes. Electrode depth was then adjusted until a typical excitatory postsynaptic potential (EPSP) was observed. Electric current was magnified by amplifier and collected by computer, then processed by DataWave software. The stimulus intensity was adjusted to produce population spike (PS) with a slope that was ≈50% of the maximum. Single-pulse test stimuli were at an interval of 30 sec during the experiment. Population spike amplitude (PSA) was recorded and the relatively percent of PSA was calculated according to the formula (PSA/ basic value).

### 3. Results

As shown in table 2, the fundamental synaptic transmission was improved by the compounds of present invention to some extent. Among them, compound Nos. 1 ((-)Δ^{5,6}-I), 2 ((+)II₁), 7 ((+)II₃), and 9 ((+)III₃) show significantly upstanding effects on synaptic transmission (P<0.05 vs. control, n=5).

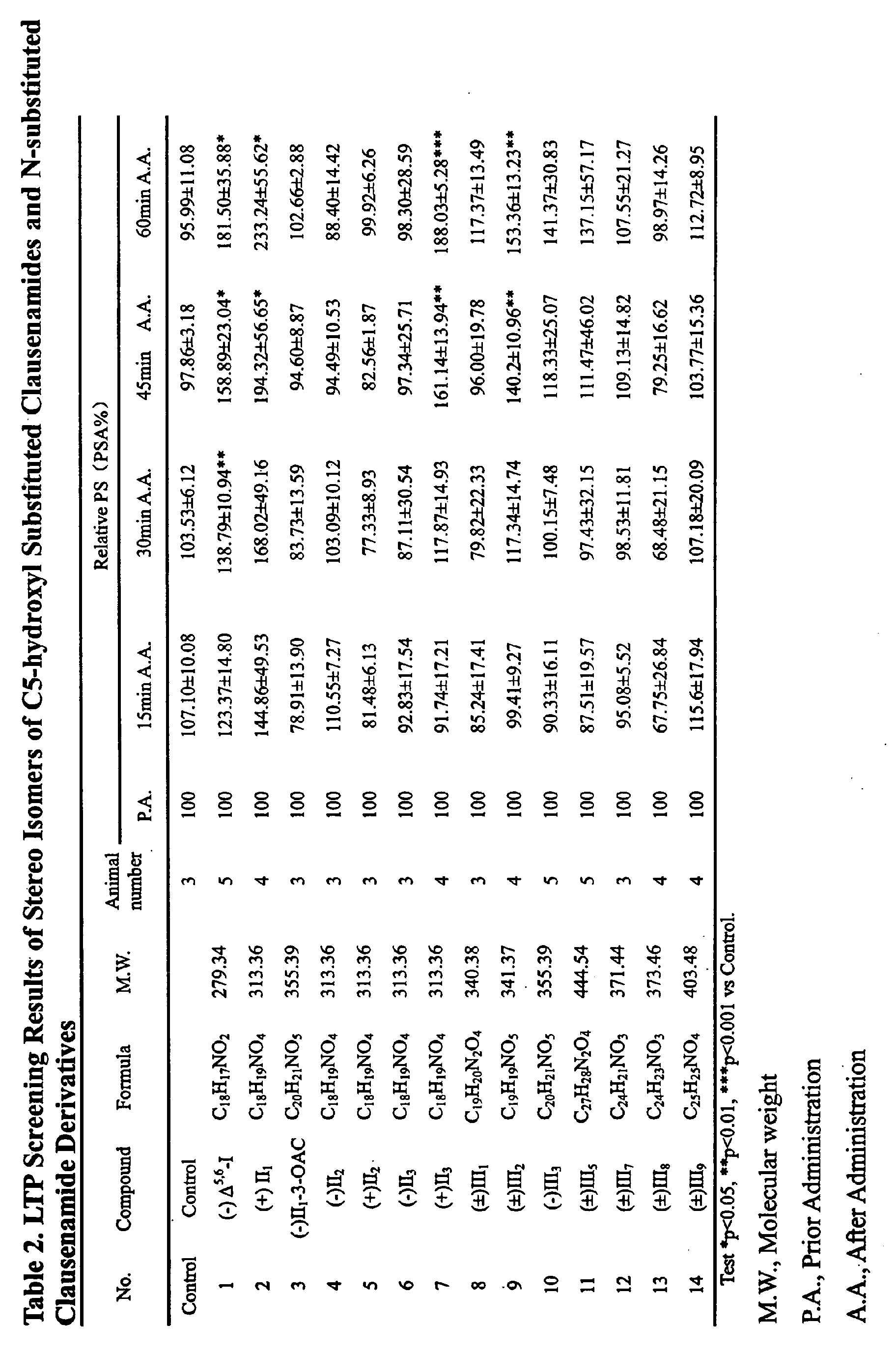

## Claims

1. An optical active C₅-hydroxyl derivative of clausenamide represented by general formula II, 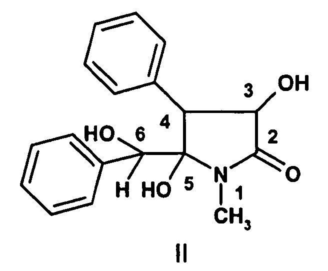 which is:
racemic II₁, configuration (3S*,4S*,5S*,6R*), or
racemic II₂, configuration (3S*,4S*,5R*,6S*), or
racemic II₃, configuration (3S*,4S*,5S*,6S*), or
optical active II₁, configuration (3S,4S,5S,6R) or (3R,4R,5R,6S), or
optical active II₂, configuration (3S,4S,5R,6S) or (3R,4R,5S,6R), or
optical active II₃, configuration (3R,4R,5R,6R) or (3S,4S,5S,6S).

2. A preparation method of the optical active C₅-hydroxyl derivative of clausenamide according to claim 1, comprising: 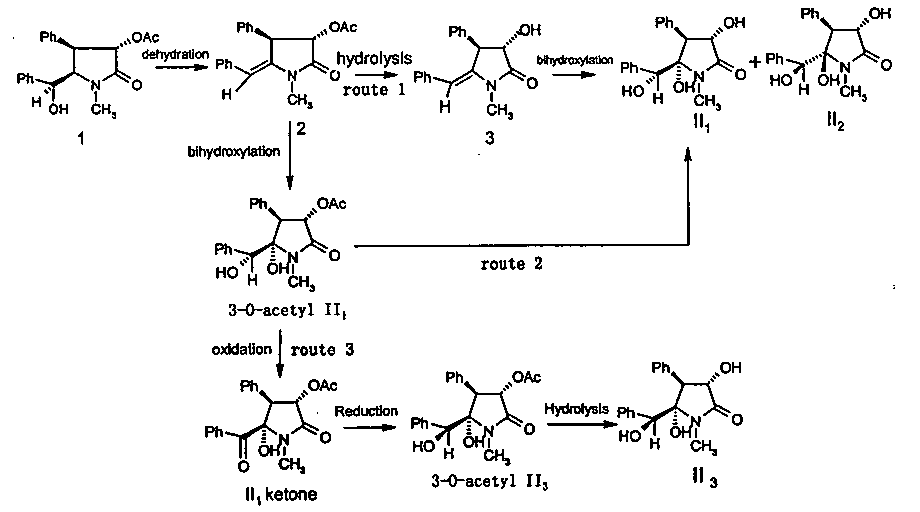
(a) dehydrolation of (rac)-3-O-acetyl-clausenamide (1) or an optical isomer thereof, the dehydralating agent may be POCl₃/Py; or to prepare the methylsulfonate of clausenamide, then cleave the methylsulfonate group with DBU;
(b) hydrolysis of (rac)-3-O-acetyl-Δ^{5,6}-clausenamide (2) or an optical isomer thereof, which can be carried out under conventional acid or base conditions;
(c) bihydroxylation of (rac)-Δ^{5,6}-clausenamide (3) or an optical isomer thereof, which can be achieved using OsO₄/NMO, KHSO₅/CH₃COCF₃, WO₃/H₂O₂;
(d) oxidation of (3S*, 4S*, 5S*, 6R*)-3-O-acetyl-5-hydroxy clausenamide (3-O-acetyl II₁) or an optical active isomer thereof, which may be carried out with oxidants such as KMnO₄/CuSO₄, MnO₂, DMSO/ClCOCOCl/TEA, DMSO/TFAA/TEA, etc;
(e) deduction of (3S*, 4S*, 5S*)-3-O-acetyl-5-hydroxy-clausenamidone (II₁ ketone) or an optical active isomer thereof, which can be carried out using various borohydrides, such as sodium borohydride or lithium tri-sec-butyl borohydride;
(f) hydrolysis of (3S*, 4S*, 5S*, 6S*)-3-O-acetoyl-5-hydroxy-clausenamide (II₃) or an optical active isomer thereof, which may be carried out using various acids or bases, or Sm/I₂/CH₃OH.

3. A N-substituted clausenamide derivative represented by general formula (III), 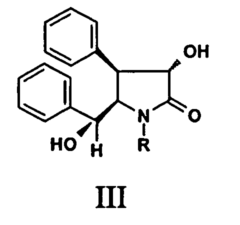 **characterized that:**
relative configuration (3S*,4R*,5R*,6S*),
R is selected from CH₂COR¹, CH₂OCH₂COR², and CH₂R³,
R¹ is selected from OH, NH₂ , C₁₋₈alkoxy, 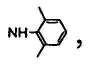 and 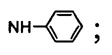
R² is selected from C₁₋₈ alkoxy, and
R³ is selected from

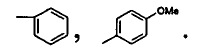

4. A preparation method of the N-substituted clausenamide derivative according to claim 3, **characterized that:**
in case R is selected from CH₂R³, which is affordable via the the reduction of N-benzyl- or N-p-methoxybenzyl-clausenamidone;
in case R is selected from CH₂COR¹ or CH₂OCH₂COR², comprising the following steps: 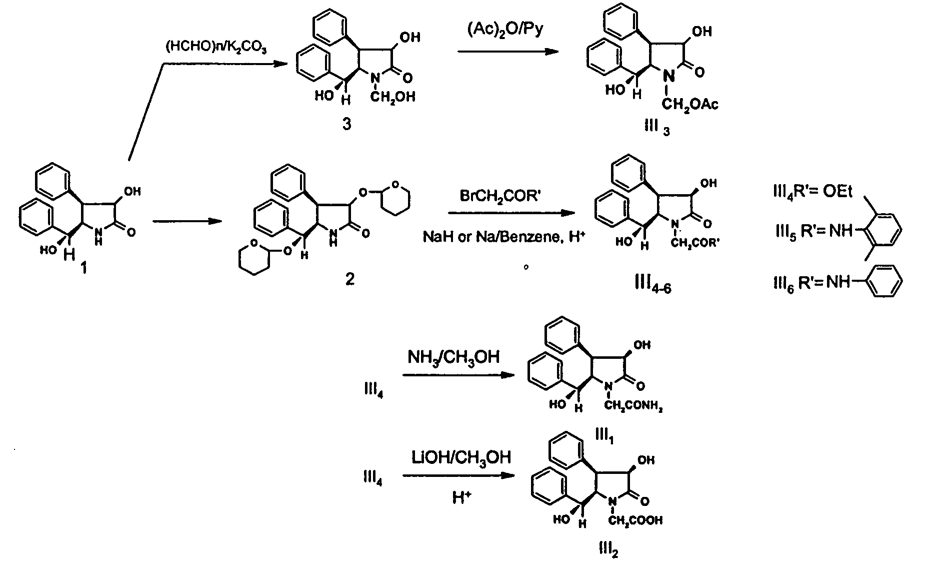
(a) reacting norclausenamide (1) with dihydropyran under the catalysis of pyridinium *p*-toluenesulfonate to give 3,6-di-O-tetrahydropyran-norclausenamide;
(b) dissolving 3,6-Di-O-tetrahydropyran-norclausenamide (2) in anhydrous benzene, adding sodium hydride, heating and adding bromoacetate, then de-protecting the protection group of tetrahydropyran to give N-(alkoxy/alkylaminocarbonylmethylene)norclausenamides;
(c) treating N-(ethoxycarbonylmethylene)norclausenamide with a largely excess amount of NH₃/CH₃OH solution to obtain N-(aminocarbonyl methylene)norclausenamide;
(d) reacting norclausenamide with paraformaldehyde and potassium carbonate to give N-(hydroxymethly)norclausenamide;
(e) reacting N-(hydroxymethly)norclausenamide with corresponding acid anhydride to prepare the corresponding N-(acyloxymethylene)-norclausenamide.

5. A pharmaceutical composition comprising a pharmacological effective amount of any compound according to claim 1 or 3 and a pharmaceutically acceptable carrier or excipient.

6. Use of a compound according to claim 1 or 3 for the preparation of medicaments as nootropic and anti-aging drugs.
